# EUROPEAN PATENT APPLICATION

(11) **EP 2 589 969 A1**
(43) Date of publication of application: **08.05.2013**
(21) Application number: 11425160.6
(22) Date of filing: 07.11.2011
(51) Int. Cl.: G01N 37/00, A61K 41/00

(54) **Physical carrier of biologically active frequencies**

(71) Applicant: Spera, Dario Maximilian, 00147 Roma (IT)
(72) Inventor: Spera, Dario Maximilian, 00147 Roma (IT)
(74) Representative: Cioncoloni, Giuliana

(57) **Abstract**

Memory device wherein biologically active frequencies are impressed i.e. recorded, storing the information therein obtained by the following steps:
*(a)* generating biologically active frequency data;
*(b)* loading the frequency data of step *(a)* in a computer and digitally processing the same therein by means of a computer programme for rendering input data into image format data;
*(c)* downloading the processed frequency data of step *(b)* into a memory device, so obtaining a conditioned memory device; *(d)* testing the biologic effect of the conditioned memory device of step *(c)* and repeating steps *(a)* to *(d)* till testing gives biologically useful effects,
whereby the memory device constitutes a physical carrier of biologically active frequencies;
and physical carrier of biologically active frequencies obtained by conditioning it thereby.

## Description

This invention relates to a physical medium wherein biologically active frequencies have been impressed i.e. recorded, so as to constitute a physical carrier of biologically active frequencies.

It is known that in medical diagnostics laboratory chemical analyses are often carried out that require some time and at times the use becomes necessary of costly machineries not always available at the moment. Moreover, it is also known that in medical therapy and for the enhancement of human performances one makes use of the chemical action of chemical compounds, particularly medicaments. In veterinary medicine and for zootechnical and agronomic enhancement too, such as for the stimulation of the production of milk, or for the preservation of organic materials, including food conservation, particularly in the refrigerator, one makes use of the chemical action of chemical compounds, particularly hormones and parasiticides. However, well known disadvantages and restrictions are connected to said chemical action.

It is the object of this invention to provide biologically active means that achieve useful biological effects without the disadvantages and the restrictions of chemical action.

By "biologically active" it is meant herein the attribute of what has an effect on biologic tissues, particularly for diagnostic and therapeutic purposes, particularly medicaments; for human enhancement; for zootechnical and agronomic applications; for food conservation; for the preservation of organic materials, such as for instance wood.

The studies are known of Nobel Prize for Medicine Luc Montagnier who has put into evidence that the appearance of diseases in living organisms corresponds to specific alterations of their electromagnetic structure. More specifically, there is an alteration of the electromagnetic field of ill cells with an alteration of the electric potential difference between the cytoplasm and the cell membrane in all pathologies. Consequently, there are electromagnetic signals, having respective frequencies, specific for each disease.

Therefore, this invention reaches the object above by exploiting biologically active frequencies.

Particularly, the biologically active frequencies may be those associated with biologically active chemical compounds, particularly medicaments, to perform the action thereof.

The biologically active frequencies may also be the opposite frequencies of those associated with harmful organisms, including bacteria and viruses, to oppose Lhe action thereof, by 'opposite frequencies' the frequencies being meant that are equal in value and phase-shifted in 180 degrees.

According to this invention, the frequencies are impressed i.e. recorded onto a target physical medium capable of retaining them as a frequency carrier in a nonvolatile way, i.e. without needing for an external power supply, and of re-emitting them when coming in contact with another physical medium by virtue of a potential difference to the latter. The other physical medium may be human skin.

It is another object of this invention to provide means for collecting and re-transmitting biologically active frequencies, particularly with the aim of amplifying them and accelerating the effect of the absorption thereof by biologic tissues.

Therefore, it is the subject-matter of this invention a memory device according to Claim 1.

It is also the subject-matter of this invention a physical medium according to Claim 2.

It is also the subject-matter of this invention a physical medium according to Claim 4.

It is also the subject-matter of this invention a physical medium according to Claim 6.

It is also the subject-matter of this invention a circuit according to Claim 14.

It is also the subject-matter of this invention a Tesla coil according to Claim 23.

It is also the subject-matter of this invention a medium according to Claim 24.

It is also the subject-matter of this invention a frequency transceiver physical medium according to Claim 25.

It is also the subject-matter of this invention a hologram according to Claim 27.

It is also the subject-matter of this invention a system according to Claim 29.

It is also the subject-matter of this invention a system according to Claim 31.

Preferred embodiments are set forth in dependent claims.

This invention will be understood based on the following disclosure, only given as a matter of example, absolutely not of restriction thereof.

This invention is a memory device wherein biologically active frequencies are impressed i.e. recorded, storing the information therein obtained by the following steps:
*(a)* generating biologically active frequency data. These ones may be both electromagnetic frequency data, including optical frequency data, and mechanical frequency data, including acoustic frequency data.

Biologically active frequencies may be both frequencies sensed from biologically active chemical compounds, particularly medicaments; hormones, including prolactin; parasiticides; antiseptics, to emulate their action; or the opposite, i.e. phase-shifted by 180°, of the frequencies sensed from harmful organisms, including bacteria and viruses, to counteract their action.

The electromagnetic frequencies of chemical compounds, e.g. of a medicament, may be sensed by means of a high resolution camera, possibly also sensible to infrared and ultraviolet radiation.

The frequencies may also be generated *de novo.* They may be applied to a person under test in a Faraday cage in the case of electromagnetic frequencies or from acoustic sound diffusers in the case of mechanical frequencies and tested in respect of their biologic effect as described below in step *(d);*
*(b)* loading the frequency data of step *(a)* in a computer and digitally processing the same therein by means of a computer programme for rendering input data into image format data, including programmes for processing photographs, videos, audios or texts, including Adobe Photoshop. In particular, through the function "Transparency" of Adobe Photoshop it is possible to 'dilute' i.e. to partialize the effect of the electromagnetic frequencies on the human body. For instance, in case the frequencies are the electromagnetic frequencies characteristic of a medicament, it is so possible to emulate the administration of half a tablet of the medicament.
*(c)* downloading the processed frequency data of step *(b)* into a memory device, including a solid-state microelectronic circuit i.e. a microchip; including a flash memory; a magnetic memory device; an optical memory device; a holographic memory device; so obtaining a conditioned memory device. Downloading the processed frequencies physically conditions the memory device. Particularly, it creates a bitmap in a microchip, featuring a specific pattern of the flow of the electrons in the microchip;
*(d)* testing the biologic effect of the conditioned memory device of step *(c)*. This may be done by putting the conditioned memory device in contact with the skin of a person. An electric potential difference arises between the skin and the memory device, making the latter to emit biologically active frequencies. Preferably the memory device is put in contact with the skin in correspondence with elective points of the human body, where the potential difference is high, particularly with the points known in the medical literature points as 'trigger points', where the electric potential difference is a maximum, so that the frequencies one wants to develop have a maximum intensity. Then the evaluation of the biologic effect of the emitted frequencies may be done by collecting response biometric data. According to a procedure, it is envisaged to test frequencies on a person on a stabilometric platform , which is a well-known apparatus for sensing the body balance performances of a person and many other data, such as blood pressure and heartbeat, or an electronic dynamometer. In a test on the stabilometric platform the betterment may be observed of postural equilibrium. In a test with a dynamometer one may observe how many kilograms and for how much time a human subject is able to push or to pull when applying the frequencies to him or her. Steps *(a)* to *(d)* are repeated adjusting the frequency emitted by the memory device, till the testing thereof achieves the desired biologically useful results, whereby the obtained memory device is a physical carrier of useful biologically active frequencies.

With a microchip memory device, this one is applied to the skin of the human body and connected through a cable to a computer through two power-supply pins and two data supply and reading pins. In this way one modifies the bitmap created in the microchip through the computer and senses the biometric results by a rheostat.

Impedance measurement of a biologic tissue provides other data to produce a useful microchip bitmap. Where there is an inflammation, a greater passage of ions is needed to have a curative effect. The bitmap is modified so that there is a lowering of the impedance of the tissue, in real time.

This invention is also a physical medium wherein biologically active radiation frequencies are impressed i.e. recorded, so that the physical medium constitutes a physical carrier of biologically active radiation frequencies.

The frequencies may be impressed by applying an electric potential difference between a target physical medium and an inventive frequency carrier memory device obtained as above or a primary biologically active physical medium, such as a medicament. The electric potential difference may be applied with the target physical medium and the memory device or primary biologically active physical medium arranged inside a Faraday cage.

Further, the frequencies may be impressed by resting an inventive memory device or a primary biologically active physical medium on a target physical medium and irradiating them with electromagnetic radiation, including infrared radiation.

The frequencies may also be impressed on a target physical medium by introducing the latter together with an inventive frequency carrier memory device, or an inventive frequency physical carrier, or any biologically active primary physical medium in a container filled with water and stirring the container.

The inventive frequency physical carrier may be composed of a plastic material, such as polyethylene (PE); polyethylene terephthalate (PET); polypropylene (PP); polyvinylchloride (PVC); polyvinyl methacrylate (PVMA); polymethyl methacrylate (PMMA); polyamide (PA) or Nylon; polyether ketone (PEK); polytetrafluoroethylene (PTFE); polycarbonate (PC) or polyesters of carbonic acid; and copolymers thereof.

The inventive carrier still further may be composed of an elastomer, such as polybutadiene rubber (PBR); natural rubber (NR) i.e. 1-4 polyisoprene; chloroprene rubber (CR) i.e. polychloroprene; silicone rubbers, such as methyl vinyl polysiloxane (VMO); fluoro elastomers and copolymers thereof.

The present invention also takes its steps from considering novel materials, such as carbon nanotubes, allowing to achieve useful and unusual properties. So, it is envisaged that the inventive physical frequency carrier includes carbon nanotubes. It has been surprisingly found by the Inventor of this application that carbon nanotubes exhibit the peculiar property that once frequencies are impressed thereon recording the frequencies therein, they show a high retentive power, i.e. they do not lose the frequencies impressed thereon in the long term under an electric voltage or under a magnetic field. The carbon nanotubes, for instance in the form of black powder, are put in a Faraday cage on a baseplate or within a cup of a material capable of carrying frequencies, such as a dielectric, for instance Mylar or quartz glass. The physical medium, which one wants to impress i.e. record the frequencies upon, is also put in the Faraday cage and an electric potential difference is applied to the baseplate or the cup. More specifically, one puts a layer of an insulating material, such as white cellulose paper, on the metal plane of the grill of the Faraday cage, with the glass quartz baseplate or cup with the carbon nanotubes thereupon. The primary physical medium which one wants to impress i.e. record the frequencies of, such as a drug or a frequency carrier microchip of this invention, is arranged within the Faraday cage put under a voltage.

It is also envisaged that the carbon nanotubes are put aside the starting substance on a conductive plate and irradiated with infrared radiation. The plate may be for instance of Mylar. It is thought that the infrared radiation excites vibration of the OH bond and of the bond of OH with C.

According to a further embodiment of this invention, the physical frequency carrier is composed of buckminster-fullerene buckyballs, particularly dispersed in another binder material, particularly a resin.

According to still a further embodiment of this invention the physical frequency carrier includes quantum dots. The latter may re-emit a frequency impressed thereupon, because they are capable of emitting electromagnetic radiation carrying the biologically active frequency information.

According to an embodiment of this invention the carrier includes a composite material, such as fibreglass.

For instance, carbon nanotubes may be dispersed in a binder material such as a resin. The binder material having the carbon nanotubes dispersed therein may be applied as a coating to an electric circuit, totally or to components thereof, such as the wires, or the power supply, or an antenna emitting frequencies. The frequency information carried by the frequency carrier composite material may be so transferred to an appliance supplied by the electric circuit, such as for instance a cellular phone.

Particularly, the circuit may be a microwave emitting circuit, such as for instance a BlueTooth device. The frequency data so are carried on a 2.45 GHz wave which is the frequency that excites the vibration of water molecules. The frequencies associated with parasiticides may be efficaciously emitted in this way for intestine.

The inventive carrier may also be composed of a siliceous material i.e. a material substantially composed of silicates, such as quartz; quartz glass; glass (substantially composed of sodium and calcium silicate); lead glass (substantially composed of siliceous glass containing lead oxide in addition to potassium and sodium oxides); steatite (a rock substantially composed of hydrated magnesium silicate); calcareous rock, including travertine silicate; natural and synthetic crystals and stones.

This invention also envisages that the frequency physical carrier is any metabolizable substance, such as sugar or salt. This is useful for bringing parasiticide characteristic frequencies to intestine.

This invention also envisages that the frequency physical carrier is composed of a metal.

It is envisaged that an inventive frequency physical carrier is embodied as a film.

According to an embodiment the frequency carrier is in the shape of a bracelet. Particularly, the bracelet may be a bracelet, for instance a fibreglass bracelet, on which there is a flexible photovoltaic cell that loads a rechargeable battery that supplies an electronic circuit emitting waves, particularly microwaves, coated with a resin having the inventive frequency carrying nanotubes disclosed above dispersed therein.

It is also envisaged that the inventive carrier is realized in the shape of a grid wherein electromagnetic frequencies of parasiticides have been impressed i.e. recorded. Such a grid arranged in a water stream is suitable for conditioning the water itself with the frequencies of the parasiticides, with benefit for the cattle which will take such frequencies by drinking the water.

Analogously an inventive carrier may have the characteristic frequency of prolactin impressed i.e. recorded therein to stimulate the production of milk.

A Tesla coil may be used supplied with biologically active frequency data by cables. The Tesla coil produces an electromagnetic field variation and elastic waves in the air consequently. A subject who finds him/herself turns out to be subjected to the biologically active frequency emitted.

The biologically active frequency data may be supplied from a frequency syntheziser, including a computer, directly connected with the power-supply of the Tesla coil.

Alternatively, a biologically active primary substance, such as a medicament, may be interposed in the power-supply system of the Tesla coil. A dielectric medium, such as quartz-glass, is interposed between the cables and the primary substance is arranged on the dielectric medium.

Moreover, a physical medium may be conditioned by immersing it in the plasma emitted by the Tesla coil supplied with the biologically active frequencies, which so are directly transmitted to the the physical medium. The physical medium may particularly be carbon nanotubes or buckyballs. Carbon nanotubes may be conditioned in the plasma of the Tesla coil placed in a quartz glass cup.

The Tesla coil is a high-voltage source producing both an electromagnetic field, including infrared radiation, and an electric flow, realizing both the methods for conditioning a physical medium according to this invention. The conditioning is direct from the Tesla coil to the target physical medium, so interferences are minimized in the conditioning, which so takes place with a minimum information loss.

The supply of biologically active frequency information both from frequency synthesizing means and from a primary biologically active substance may be used.

This invention is also a frequency transceiver as a means for picking-up and re-transmitting biologically active frequencies, particularly with the aim of amplifying them and accelerating the effect of the absorption thereof by biologic tissues. This frequency transceiver is formed as an elongate material shaped as an 'eight' figure without crossings. By 'elongate material' it is meant herein a material in a shape having a length and having its cross-section small with respect to its length, including a wire, a strip, a hollow tube. The material is preferably a dielectric material, particularly Mylar.

A potential difference is to be applied thereto, such as by means of a battery, a photovoltaic cell or a direct-current transformer or a Tesla coil in the case of the hollow tube, wherein the latter becomes a plasma tube. The applied electric potential difference is to polarize the material.

Such frequency transceiver is useful once put in proximity or in direct contact with a subject, such as a human subject, an animal or a plant, preferably at elective points, for example elective points on the skin of a human subject. The frequency transceiver picks up the frequencies falling in proximity with the subject carrying it.

The frequency transceiver may also be useful jointly with a medicament (i.e. a biologically active principle carrier) in proximity with a human subject. The frequency transceiver picks-up the frequencies associated with the medicament and re-transmits them onto the human subject.

This invention also envisages conditioning targets from starting physical carriers with infrared radiation. More particularly, this invention envisages recording the characteristic frequencies of a starting physical medium by impressing them on a transparent physical carrier. The so impressed frequency carrier transparent material is interposed between a photon source and a target. The latter will be undergo the effect of the frequencies carried by the impressed transparent material.

So, frequency carrier transparent media may be used to condition plants in greenhouses availing oneself of solar radiation. So the action may be performed on plants for instance of pesticides, or growth hormones.

This invention also envisages creating holograms of memory devices or physical carriers recording biologically active frequencies. The holograms are three-dimensional planar holograms generated starting from a real object. The holograms comprise information stored on a surface: this information represents all the characteristics of the starting object, in the case of the inventive carriers the frequencies carried thereby too. So, the holograms will have the same effect as the original microchip memory devices or physical carriers.

This invention is also a system including a central computer, including an operating system, and peripheral memory devices. A mother-folder is created in the operating system, suitable for downloading the frequencies carried by an inventive memory device therein. The system also includes communication means by which the central computer is in functional communication with the peripheral memory devices.

This invention envisages that the peripheral memory devices store empty daughter-folders created therein by copying and pasting the mother-folders created in the central computer.

When a bitmap of a biologically active frequency as disclosed above is downloaded in the mother-folder, the biologically active effect of that bitmap is automatically transferred onto the microchip memory device having the daughter-folder therein, and the microchip with the daughter-folder turns out to emit the same biologically effective frequencies as those of the mother-folder when applied on human skin. Without willing to be tied to any theory, it is believed that this is a quantum entanglement effect.

The frequency information content carried by a microchip so obtained may be impressed onto one or more target carrier physical media as taught above, constituting peripheral frequency physical carriers.

Such an empty folder copy-and-paste procedure may be repeated for a number of microchips and so it is possible to correspondingly impress frequencies on a number of microchips with a single operation affecting a single mother-folder, but a plurality of daughter-folders.

So the advantage is also achieved that the bitmap may be upgraded at any time.

The inventive system may further include a machinery, in functional communication with the central computer, suitable for detecting the electric potential difference between the cellular membrane and the cytoplasm, and storing a database of biologically active frequencies. On detecting a difference falling out of a healthy range, the machinery automatically communicates one or more frequencies capable of restoring the healthy electromagnetic field of the cell, i.e. of bringing the potential difference to the ideal one back, to the central computer, selecting the frequencies from the database.

The communication may take place as follows. If a bit in a mother-folder is modified, correspondingly there is a variation in the electromagnetic field and in the wavelength emitted by a carrier storing a daughter-folder pasted therefrom. This variation may be sensed by a spectrometer. If the daughter-folder is applied on the photovoltaic cell, a variation will be observed in the voltage emitted thereby. So the variation is detectable of the field or the radiation wavelength. Inserting frequency data involves modifying bits in the bitmap of the mother-folder. Sensing such variations enables one to create a communication protocol between the mother-folder and the daughter-folders and among the daughter-folders. This enables a communication between a patient user owning a frequency carrier and a physician at the central computer.

This invention has been disclosed having reference to exemplifying embodiments thereof, but variations may be made thereto without departing from the scope of protection thereof, which only is defined by the appended claims.

## Claims

1. Memory device wherein biologically active frequencies are impressed i.e. recorded, storing the information therein obtained by the following steps:
*(a)* generating biologically active frequency data, including electromagnetic frequency data, including optical frequency data, and including mechanical frequency data, including acoustic frequency data, which frequencies are synthesized or sensed, including frequencies sensed from biologically active chemical compounds, including medicaments; hormones, including prolactin; parasiticides; antiseptics; and including frequencies synthesized as the opposite frequencies of the frequencies sensed from harmful organisms, including bacteria and viruses;
*(b)* loading the frequency data of step *(a)* in a computer and digitally processing the same therein by means of a computer programme for rendering input data into image format data, including programmes for processing photographs, videos, audios or texts, including Adobe Photoshop;
*(c)* downloading the processed frequency data of step *(b)* into a memory device, including a solid-state microelectronic circuit i.e. a microchip; including a flash memory; a magnetic memory device; an optical memory device; a holographic memory device; so obtaining a conditioned memory device;
*(d)* testing the biologic effect of the conditioned memory device of step *(c)* and repeating steps *(a)* to *(d)* till testing gives biologically useful effects,
whereby the memory device constitutes a physical carrier of biologically active frequencies.

2. Physical medium wherein the biologically active frequencies are impressed carried by a memory device of Claim 1, so that it constitutes a physical carrier of biologically active frequencies.

3. Physical medium of Claim 2 wherein biologically active frequencies are impressed by applying an electric potential difference between a target physical medium and a memory device of Claim 1 or a primary biologically active physical medium.

4. Physical medium obtained by introducing a target physical medium together with a memory device of Claim 1, or a physical medium of Claim 2, or any biologically active primary physical medium in a container filled with water and stirring the container.

5. Physical medium of Claim 2, wherein said electric potential difference is applied with said target physical medium and said memory device or primary biologically active physical medium arranged inside a Faraday cage.

6. Physical medium wherein biologically active frequencies are impressed by resting a memory device according to Claim 1 or a primary biologically active physical medium on a target physical medium and irradiating them with electromagnetic radiation, including infrared radiation.

7. Physical medium of Claim 2, composed of a plastic material, including polyethylene (PE); polyethylene terephthalate (PET); polypropylene (PP); polyvinylchloride (PVC); polyvinyl methacrylate (PVMA); polymethyl methacrylate (PMMA); polyamide (PA) or Nylon; polyetherketone (PEK); polytetrafluoroethylene (PTFE); polycarbonate (PC) or polyesters of carbonic acid; and copolymers thereof.

8. Physical medium of Claim 2, composed of an elastomer, including polybutadiene rubber (PBR); natural rubber (NR) i.e. 1-4 polyisoprene; chloroprene rubber (CR) i.e. polychloroprene; silicone rubbers, including methyl-vinylpolysiloxane (VMO); fluoroelastomers and copolymers thereof.

9. Physical medium of Claim 2, composed of carbon nanotubes.

10. Physical medium of Claim 2, including buckminster-fullerene buckyballs.

11. Physical medium of Claim 2, including quantum dots.

12. Physical medium of Claim 2, composed of a composite material.

13. Physical medium of Claim 12 wherein said composite material includes carbon nanotubes dispersed therein.

14. Electric circuit partially or totally coated with the physical medium of Claim 12.

15. Electric circuit of Claim 14 being a circuit including a component emitting waves, including microwaves, including a BlueTooth device, and including infrared radiation frequencies, including a light-emitting-diode (LED).

16. Physical medium of Claim 2, composed of a siliceous material, including quartz; quartz glass; glass; lead glass; steatite; calcareous rock, including travertine silicate; natural and synthetic crystals and stones.

17. Physical medium of Claim 2 composed of a metabolizable substance, including sugar and salt.

18. Physical medium of Claim 2 composed of a metal.

19. Physical medium of Claim 2 in the shape of a film.

20. Physical medium of Claim 19 in the shape of a bracelet.

21. Physical medium of Claim 20, whereon there is a flexible photovoltaic cell that loads a rechargeable battery that supplies an electronic circuit coated with a resin having the inventive frequency carrying nanotubes disclosed above dispersed therein.

22. Physical medium of Claim 2 in the shape of a grid.

23. Tesla coil endowed with means for supplying biologically active frequencies thereto.

24. Physical medium loaded with biologically active frequency data by means of the Tesla coil of Claim 23.

25. Frequency transceiver physical medium formed as an elongate material, including a dielectric, including Mylar, shaped as an 'eight' figure without crossings endowed with means for applying a potential difference thereto.

26. Physical medium of Claim 2 being a transparent medium.

27. Hologram of a memory device according to Claim 1.

28. Hologram of a physical carrier according to Claim 2.

29. System including a central computer, including an operating system, and peripheral memory devices; a mother-folder being created in said operating system, suitable for downloading the frequencies carried by an inventive memory device therein; and including communication means whereby said central computer is in functional communication with said peripheral memory devices.

30. System of Claim 26 wherein said peripheral memory devices store empty daughter-folders created therein by copying and pasting said mother-folders created in said central computer.

31. System of Claim 27 further including a machinery, in functional communication with said central computer, suitable for detecting the electric potential difference between the cellular membrane and the cytoplasm, and storing a database of biologically active frequencies; which machinery, on detecting an electric potential difference falling out of a healthy range, automatically communicates one or more frequencies capable of restoring the healthy electromagnetic field of the cell, i.e. of bringing the potential difference to the ideal one back, to said central computer, selecting the frequencies from said database.
